# EUROPEAN PATENT APPLICATION

(11) **EP 2 269 972 A1**
(43) Date of publication of application: **05.01.2011**
(21) Application number: 09725667.1
(22) Date of filing: 26.03.2009
(51) Int. Cl.: C07C 50/36, C07C 49/747, C07C 49/753, A61K 31/122, C12N 1/21, C12N 15/76, C12N 9/10, A61P 35/00, C12R 1/47

(54) **OVIEDOMYCIN DERIVATIVES, METHOD FOR OBTAINING SAME AND USE THEREOF**

(30) Priority: 28.03.2008 ES 200800982
(71) Applicant: Universidad De Oviedo, 33006 Oviedo (ES)
(72) Inventor: MÉNDEZ FERNÁNDEZ, Carmen, 33006 Oviedo (ES); LOMBÓ BRUGOS, Felipe, 33006 Oviedo (ES); FERNÁNDEZ BRAÑA, Alfredo, 33006 Oviedo (ES); SALAS FERNÁNDEZ, José Antonio, 33006 Oviedo (ES)
(74) Representative: Illescas, Manuel
(86) International application number: PCT/ES2009/070076
(87) International publication number: WO 2009/118443

(57) **Abstract**

Oviedomycin derivatives, method for obtaining same and use thereof. This invention relates to oviedomycin derivatives obtained by fermentation of recombinant bacterial strains. The invention also relates to the methods used to obtain the recombinant strains and to produce the oviedomycin derivatives. The invention further relates to bacterial strains that can be used to produce oviedomycin derivatives. Finally, said oviedomycin derivatives are applicable in the field of the human health, specifically to produce drugs for the treatment of tumour diseases.

## Description

The invention relates to the pharmaceutical field and specifically relates to compounds applicable in oncology, with chemical structure derived from oviedomycin and which is obtained by the fermentation of microorganisms.

### STATE OF THE ART

Oviedomycin (OVM) is an aromatic polyketide of angucyclinone type produced by *Streptomyces antibioticus* ATCC 11891. The chemical structure of OVM (Fig. 1) and the biosynthetic genes responsible for its biosynthesis are known (J. Nat. Prod. 2002, 65, 779-782; Chembiochem. 2004, 5, 1181-1187). Angucyclinones are natural products that belong to the family of angucyclines, characterized by having a quinone system in the C ring of a tetracyclic benzanthracene system (Nat. Prod. Rep. 1992, 9, 103-137; Nat. Prod. Rep. 1999, 16, 425-484). Anti-tumour activity has been described for several angucyclines, such as urdamycin and 6-hydroxy-tetrangulol (J. Antibiot. 1986, 39, 1657-1669; J. Antibiot. 1998, 51, 79-81).

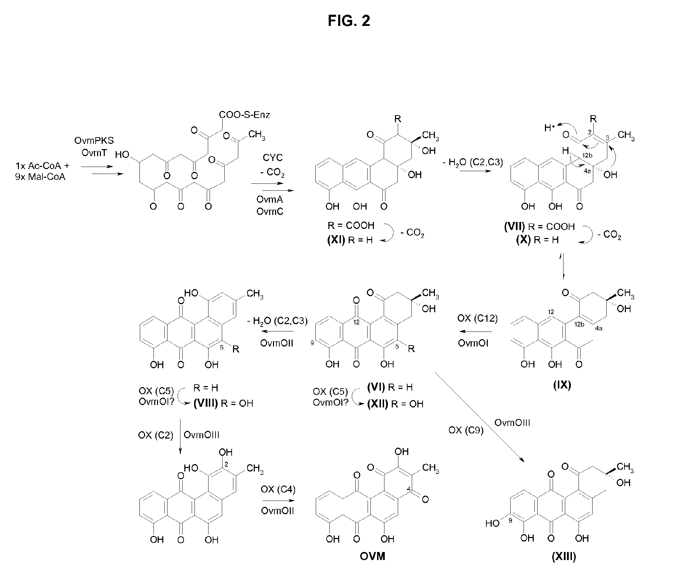

The biosynthesis of OVM starts with the generation of a linear polyketide with 20 carbon atoms, synthesized by a polyketide synthase and a ketoreductase (OvmPKST, Fig. 2). This linear polyketide undergoes a series of cyclizations carried out by the aromatase OvmA and the cyclase OvmC *(*Chembiochem. 2004, 5, 1181-1187). The grouping of genes responsible for OVM biosynthesis contains three genes which encode FAD-dependent oxygenases (*ovmOI, ovmOII, ovmOIII*)*.* These oxygenases are responsible for the introduction of keto groups in positions C-4 and C-12 (part of the quinone systems of the A and C rings, respectively) and of the hydroxyl group in position C-2.

There is currently a great need for new anti-tumour agents, with improved activity, with less undesirable secondary effects and with greater selectivity, in comparison with the drugs currently in use. The pharmaceutical industry has traditionally developed new drugs through two fundamental channels: (1) the search for new natural products, and (2) the synthesis and/or chemical modification of certain compounds. These methods continue to be useful, but they usually require very considerable investments in resources

(time, money, energy), as it is normally necessary to analyse thousands of products to find a new promising compound. The development of recombinant DNA technology has opened an interesting field of research to generate new bioactive compounds with the manipulation of genes involved in the biosynthesis of anti-tumour agents, mainly bacteria from the group of actinomycetes *(*Trends Biotechnol. 2001, 19, 449-456; J. Mol. Microbiol. Biotechnol. 2005, 9, 77-85; Curr. Opin. Drug Discov. Devel. 2005, 8, 748-756; J. Ind. Microbiol. Biotechnol. 2006, 33, 560-568; Curr. Opin. Microbiol. 2006, 9, 252-260). These techniques can also be used to improve the production of already known natural compounds, since the natural strains usually produce low concentrations of the metabolite of interest.

### DESCRIPTION OF THE INVENTION

The present invention provides new bacterial strains that produce compounds of angucycline type: some of these compounds are new, whilst other compounds are already known. Several of these compounds have a greater anti-tumour activity *in vitro* in comparison with the activity shown by OVM. These bacterial strains are obtained by introducing certain additional nucleic acids in strains of *Streptomyces* spp. that are not angucycline producers, in particular of *Streptomyces albus.* Said nucleic acids encode enzymatic activities involved in the biosynthesis of OVM, and can be obtained from *Streptomyces albus* ATCC 11891 or from any other OVM producing microorganism.

Nucleic acids can be introduced in *Streptomyces* spp. by transformation of protoplasts, conjugation, or other known methods (such as those described in Practical Streptomyces genetics, The John Innes Foundation, Norwich, Great Britain, 2000), so that the nucleic acids are replicable in the organism, either in the form of extrachromosomal element or integrated in the organism chromosome.

The bacterial strains of this invention can be cultivated in any suitable medium, in conditions that allow their growth, as described in J. Nat. Prod. 2002, 65, 779-782; Chembiochem. 2004, 5, 1181-1187. After several days' incubation these cultures contain high cell levels (mycelium), together with a mixture of compounds, including OVM derivatives. Next, the cultures undergo processes to separate the liquid phase (supernatant) and a solid phase (mycelium). Then the two phases undergo, separately, various methods that may include extraction with various organic solvents and different types of chromatographies (such as HPLC, high performance liquid chromatography), in order to obtain the OVM derivatives in the form of pure compounds. The OVM derivatives have anti-tumour and antibiotic activity.

Likewise, the present invention provides compounds characterized in that they have the following formulas (I), (II), (III), (IV) and (V): 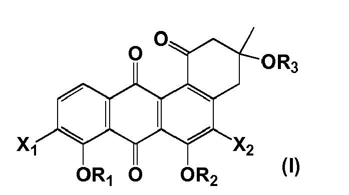 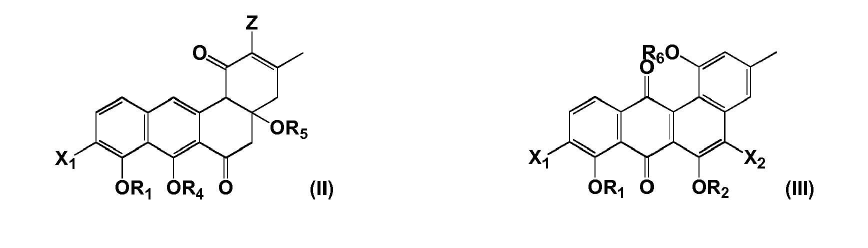 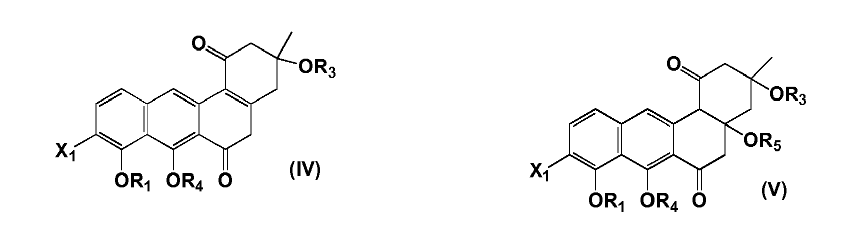 where
R₁, R₂, R₃, R₄, R₅ and R₆ are, each one and independently, hydrogen or a protector group. The protector group may consist of an alkyl group, a cycloalkyl group, a heterocyclic cycloalkyl group, a hydroxyalkyl group, a halogenated alkyl group, an alkoxyalkyl group, an alkenyl group, an alkynyl group, an aryl group, a heterocyclic aryl group, an alkylaryl group, an ester group, a carbonate group, a carboxylic acid group, an aldehyde group, a ketone group, a urethane group, a silyl group, a sulfoxide group or a combination thereof.
X₁ and X₂ are, each one and independently, hydrogen, a hydroxyl group or an -OR₁ group, where R₁ is a protector group according to the previous definition,
Z is hydrogen or a group chosen from the following groups: a carboxylic acid group, an ester group, a carbonate group, a carbamate group, a urethane group, a hydroxyl group, an alkyl group, a cycloalkyl group, a heterocyclic cycloalkyl group, a hydroxyalkyl group, a halogenated alkyl group, an alkoxyalkyl group, an alkenyl group, an alkynyl group, an aryl group, a heterocyclic aryl group, an alkylaryl group, an aldehyde group or a ketone group.

In particular, the present invention provides, among others, the compounds with the following formulas (VI, VII, VIII, IX, X, XI, XII, XIII):

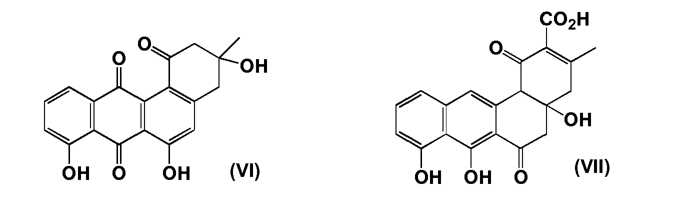

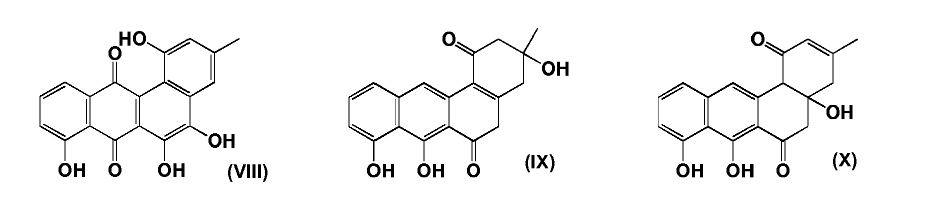

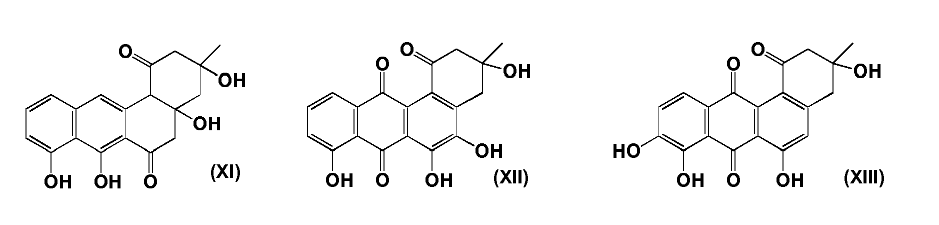

The present invention provides strains for obtaining new compounds, not previously obtained [formulas (VII), (VIII), (IX) and (XIII)]. Furthermore, the present invention provides new strains for obtaining already described compounds [formulas (VI), (X), (XI) and (XII)].

The compounds of the invention are tumour growth inhibitors and are, therefore, useful in the treatment of cancer.

In this way, an object of the present invention are the pharmaceutical compositions which comprise an effective quantity of a compound of any of formulas (I), (II), (III), (IV), (V), (VI), (VII), (VIII), (IX), (X), (XI), (XII) or (XIII) or a pharmaceutically acceptable salt or solvate thereof together with a pharmaceutically acceptable excipient.

A further object of the present invention is the use of a compound of any of formulas (I), (II), (III), (IV), (V), (VI), (VII), (VIII), (IX), (X), (XI), (XII) or (XIII) or a pharmaceutically acceptable salt or solvate thereof in the manufacturing of a drug.

A further object of the present invention is the use of a compound of any of formulas (I), (II), (III), (IV), (V), (VI), (VII), (VIII), (IX), (X), (XI), (XII) or (XIII) or a pharmaceutically acceptable salt or solvate thereof to inhibit the growth of a tumour.

As used here "inhibit" means to reduce, make slower, or stop. Therefore, a compound of this invention may reduce, make slower or stop the growth of a tumour cell. As used here, "growth" means increase in size, or proliferation, or both. Therefore, a compound of this invention may inhibit the increase in size of a tumour cell and/or may prevent the tumour cell from dividing and increase the number of tumour cells. A "tumour cell" is a cell that constitutes a neoplasm (new growth), which may be cancerous (malignant) or non-cancerous (benign). A cancerous tumour cell may invade the normal tissue surrounding it and the blood/lymphatic vessels and form metastasis in tissue far from the original tumour. In contrast, a non-cancerous tumour cell may grow and compress the adjacent normal tissues but it cannot invade normal tissues and blood/lymphatic vessels and neither can it form metastasis in tissues far from the original tumour.

A further object of the present invention is the use of a compound of any of formulas (I), (II), (III), (IV), (V), (VI), (VII), (VIII), (IX), (X), (XI), (XII) or (XIII) or pharmaceutically acceptable salt or solvate thereof to treat cancer.

A further object of the present invention is the use of a compound of any of formulas (I), (II), (III), (IV), (V), (VI), (VII), (VIII), (IX), (X), (XI), (XII) or (XIII) or a pharmaceutically acceptable salt or solvate thereof in the manufacturing of a drug with anti-tumour activity.

A further object of the present invention is the use of a compound of any of formulas (I), (II), (III), (IV), (V), (VI), (VII), (VIII), (IX), (X), (XI), (XII) or (XIII) or a pharmaceutically acceptable salt or solvate thereof in the manufacturing of a drug for the treatment of cancer.

A further object of the present invention is a method of treatment of a subject, including a human being, diagnosed with cancer, which consists of treating said subject with a therapeutically effective quantity of a compound of any of formulas (I), (II), (III), (IV), (V), (VI), (VII), (VIII), (IX), (X), (XI), (XII) or (XIII) or a pharmaceutically acceptable salt or solvate.

As used here, a "subject" may include pets (for example, cats, dogs, etc.), farm animals (for example, cows, horses, pigs, sheep, goats, etc.), laboratory animals (for example, mice, rabbits, guinea pigs, etc.) and birds. Preferably, the subject is a mammal such as a primate and, with greater preference, a human being.

In general, an "effective quantity" of a compound is that quantity necessary to achieve the desired result. For example, the effective quantity of a compound of the present invention treats cancer by inhibiting the growth of cells that constitute the tumour, so that it prevents the invasion of normal tissues and blood/lymphatic vessels by the tumour cells and, therefore, prevents metastasis. Examples of cancers that can be treated include, but are not limited to, lung, colon, ovarian, prostate, testicular, melanoma, kidney, breast, central nervous system and leukaemia. The expression "pharmaceutically acceptable composition" consists of a biologically suitable material, i.e. that the material can be administered to the subject without causing him/her/it substantially harmful biological effects.

The doses or quantities of the compounds of the invention must be sufficiently large to produce the desired effect. However, the dose must not be as large as to cause adverse secondary effects, for example unwanted cross reactions, anaphylactic reactions and such like. Generally, the dose will vary with age, condition, sex and degree of the subject's disease and can be determined by any person skilled in the art. The dose can be adjusted by each doctor, based on the clinical condition of the subject involved. The dose, dosage regime and administration route may vary.

Any of the compounds of the invention can be used therapeutically forming part of an acceptable pharmaceutical composition. Any person skilled in the art can create acceptable pharmaceutical compositions, which may consist of sterile solutions in water, saline solutions or solutions buffered to physiological pH. Any of the compounds of the invention may be prepared in the form of pharmaceutical composition. The pharmaceutical compositions may include various carrier agents, thickeners, buffers, preservatives, surfactants, and others, in addition to the compound of the invention. The pharmaceutical compositions may further include active ingredients such as antimicrobial agents, anti-inflammatory agents, anaesthetic agents, etc.

The compounds of the invention may be administered to the subject in several different ways, depending on whether one wants the treatment to be local or systemic, and depending on the area to be treated. Thus, for example, a compound of the present invention may be administered in the form of ophthalmic solution, for application on the eye surface. Furthermore, a compound may be administered to a subject by vaginal, rectal, intranasal, oral, by inhalation, or by parenteral route, whether intradermal, subcutaneous, intramuscular, intraperitoneal, intrarectal, intra-arterial, intralymphatic, intravenous, intrathecal and intratracheal. Parenteral administration, if used, is generally performed by injection. The solutions for injection can be prepared in various ways, such as solutions or liquid suspensions, solid forms suitable for being dissolved or placed in suspension before the injection, or as emulsions. Other forms of parenteral administration use systems of slow or sustained release, so that a constant dose is achieved (see, for example, patent US 3,710,795). The preparations for parenteral administration include sterile aqueous or non-aqueous solutions, suspensions, and emulsions, and they may also contain buffers and diluent additives and others. Examples of non-aqueous solvents are: propylene glycol, polyethylene glycol, vegetable oils such as olive oil, and organic esters for injection such as ethyl oleate. Examples of aqueous solvents are: water, alcohol-aqueous solutions, emulsions or suspensions, including saline and buffer solutions. Examples of parenteral vehicles are: sodium chloride solution, Ringer's dextrose, sodium chloride and dextrose, etc. Preservatives and other additives may also be present, such as, for example, antimicrobial agents, anti-oxidants, chelating agents, inert gases, etc. The formulations for topical administration may include creams, lotions, gels, drops, suppositories, sprays, liquids and powders. Certain conventional pharmaceutical carriers, aqueous bases, oily bases or, in powder, thickeners, etc. may also be necessary. The compositions for oral administration may include powders or granules, suspensions or solutions in water or non-aqueous medium, capsules or tablets. It may be desirable to include thickening, flavouring, diluent, emulsifying, dispersant agents, etc.

For the purposes of the present invention and its description, the term oviedomycin "derivative" must be interpreted as compound covered by any of formulas (I), (II), (III), (IV) or (V). Likewise, the term oviedomycin "prodrug" must be interpreted for the purposes of the present invention and description thereof, as any compound that releases, when circulating in the blood or entering the cell, oviedomycin or a derivative, in accordance with any of formulas (I), (II), (III), (IV) or (V), thereof.

### BRIEF DESCRIPTION OF THE DRAWINGS

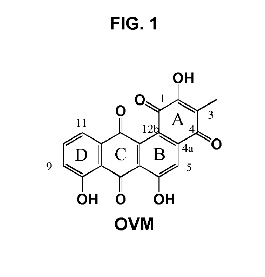

Fig. 1. Chemical structure of OVM.
Fig. 2. Biosynthesis of OVM and derivatives. Abbreviations: Ac-CoA (acetyl-coenzyme A), Mal-CoA (malonyl-coenzyme A), OX (oxygenation), CYC (cycling), -CO₂ (decarboxylation), -H₂O (dehydration), OVM (oviedomycin).
Fig. 3A. Genetic organization of the groups of genes responsible for OVM biosynthesis.
Fig. 3B. Plasmids used in this work, showing their genetic composition. The black triangles represent the promoter P*_{ermE}.
Fig. 4A, Fig. 4B, Fig. 4C, Fig. 4D. HPLC analysis of culture extracts of *Streptomyces albus* strains containing plasmids pFL1031 (Fig. 4A), pFL1033 (Fig. 4B), pFL1030 (Fig. 4C) and pFL1146 (Fig. 4D). Peak identifier: VI = rabelomycin; VII = prejadomycin 2-carboxylate; VIII = 5-hydroxy-dehydro-rabelomycin; IX = 4a,12b-dehydro-UWM6; X = prejadomycin; XI = UWM6; XII = 5-hydroxy-rabelomycin; XIII = 9-hydroxy-rabelomycin.
Fig. 5. Chemical structures of oviedomycin (OVM), rabelomycin [formula (VI)], prejadomycin-2-carboxylate [formula (VII)], 5-hydroxy-dehydro-rabelomycin [formula (VIII)], 4a,12b-dehydro-UWM6 [formula (IX)], prejadomycin [formula (X)], UWM6 [formula (XI)], 5-hydroxy-rabelomycin [formula (XII)], and 9-hydroxy-rabelomycin [formula (XIII)].

### EXPLANATION OF A PREFERRED EMBODIMENT

For a better understanding of the present invention, the following examples are given, described in detail, which must be understood without limiting the scope of the invention.

The following examples use DNA manipulation techniques well known in the state of the art, such as those described by Sambrook et al. (Molecular Cloning, a Laboratory Manual, 2nd ed., Cold Spring Harbor Laboratory Press, New York, 1989), and by Kieser et al. (Practical Streptomyces genetics, The John Innes Foundation, Norwich, Great Britain, 2000).

### Example 1. Construction of plasmid FL1028.

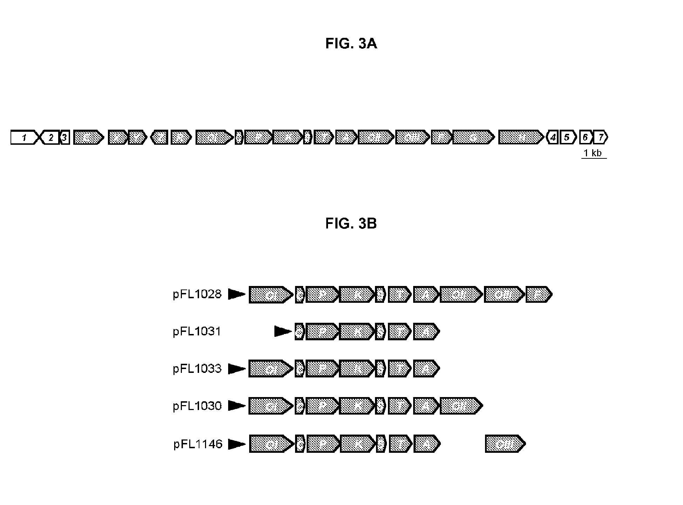

For the construction of plasmid pFL1028, the cosmid cosAB4 (J. Nat. Prod. 2002, 65, 779-782; Chembiochem. 2004, 5, 1181-1187) was digested with the restriction enzymes *Bfu*I and *Sgf*I, and a DNA fragment of 10906 bp was obtained whose ends were blunted. This DNA fragment was subcloned in pEM4A (Mol. Microbiol. 1998, 28, 1177-1186), which had been previously digested with EcoRI and its ends blunted, generating the pFL1028 plasmid. In this plasmid, the *ovmOI, ovmC, ovmP, ovmK, ovmS, ovmT, ovmA, ovmOII, ovmOIII* and *ovmF* genes are expressed under the control of the promoter *ermE*p* (Fig. 3).

**Example 2. Obtaining the bacterial strain *Streptomyces albus* (pFL1030).**

The strain *Streptomyces albus* (pFL1030) was generated from *Streptomyces albus* J1074 (J. Gen. Microbiol. 1980, 116, 323-334) by the introduction of plasmid pFL1030. For the construction of plasmid pFL1030, plasmid pFL1028 was digested with the restriction enzymes *Bfu*I and *Kpn*I*,* and a DNA fragment was obtained of 8966 bp. This DNA fragment (after blunting its ends) was cloned in pEM4A, which had been previously digested with *Eco*RI and its ends blunted, generating plasmid pFL1030. In this plasmid, the *ovmOI, ovmC, ovmP, ovmK, ovmS, ovmT, ovmA* and *ovmOII* genes are expressed under the control of the promoter P**_{ermE}* (Fig. 3).

The strain Streptomyces albus (pFL1030) was deposited on 26/02/2008 in the Colección Española de Cultivos Tipo (CECT) (Spanish Type Culture Collection), University of Valencia, Campus de Burjassot, 46100 Burjassot (Valencia, Spain) with access number CECT 7381.

### Example 3. Obtaining the bacterial strain Streptomyces albus (pFL1031).

The strain *Streptomyces albus* (pFL1031) was generated from *Streptomyces albus* J1074 (J. Gen. Microbiol. 1980, 116, 323-334) by the introduction of plasmid pFL1031. For the construction of plasmid pFL1031, plasmid pFL1028 was digested with the restriction enzymes *Sph*I and *Xho*I to obtain a fragment of 5487 bp. This DNA fragment (after blunting its ends) was cloned at pEM4A, which had been previously digested with *Eco*RI and its ends blunted, generating plasmid pFL1031. In this plasmid, the *ovmC, ovmP, ovmK, ovmS, ovmT,* and *ovmA* genes are expressed under the control of the promoter P**_{ermE}* (Fig. 3).

The strain Streptomyces albus (pFL1031) was deposited on 26/02/2008 in the Colección Española de Cultivos Tipo (CECT) (Spanish Type Culture Collection), University of Valencia, Campus de Burjassot, 46100 Burjassot (Valencia, Spain) with access number CECT 7380.

### Example 4. Obtaining the bacterial strain Streptomyces albus (pFL1033).

The strain *Streptomyces albus* (pFL1033) was generated from *Streptomyces albus* J1074 (J. Gen. Microbiol. 1980, 116, 323-334) by the introduction of plasmid pFL1033. For the construction of plasmid pFL1033, plasmid pFL1028 was digested with the restriction enzymes *Bfu*I and *Xho*I to obtain a fragment of 6804 bp. This DNA fragment (after blunting its ends) was cloned in pEM4A, which had been previously digested with *Eco*RI and its ends blunted, generating plasmid pFL1033. In this plasmid, the *ovmOI, ovmC, ovmP, ovmK, ovmS, ovmT,* and *ovmA* genes are expressed under the control of the promoter P**_{ermE}* (Fig. 3)

### Example 5. Obtaining the bacterial strain Streptomyces albus (pFL1146).

The strain *Streptomyces albus* (pFL1146) was generated from *Streptomyces albus* J1074 (J. Gen. Microbiol. 1980, 116, 323-334) by the introduction of plasmid pFL1146. For the construction of plasmid pFL1146, plasmid pFL1028 was digested with the *Sal*I restriction enzyme to obtain a fragment of 2541 bp which contained the *ovmOIII* gene. This DNA fragment was cloned in the *SalI* site of pUC18, generating plasmid pFL1142. This plasmid was digested with *Hin*dIII, and its ends blunted and later digested with *Xba*I*,* to obtain a fragment containing the *ovmOIII* gene which was cloned at the sites *Xba*I and *Pst*l (this last site blunt) of the pIAGO vector *(*Mol. Microbiol. 1998, 28, 1177-1186), generating plasmid pFL1144. Finally, an *Eco*RI-*Hind*III fragment was obtained (with blunt ends) from pFL1144, containing *ovmOIII* under the control of the promoter P*_{ermE}*, which was cloned at the Hindlll site (blunted) of pFL1033, generating plasmid pFL1146. This plasmid contains the *ovmI, ovmC, ovmP, ovmK, ovmS, ovmT,* and *ovmA* genes under the control of the promoter P**_{ermE}*, and the *ovmOIII* gene under the control of the promoter P*_{ermE}* (Fig. 3).

The strain Streptomyces albus (pFL1146) was deposited on 26/02/2008 in the Colección Española de Cultivos Tipo (CECT) (Spanish Type Culture Collection), University of Valencia, Campus de Burjassot, 46100 Burjassot (Valencia, Spain) with access number CECT 7379.

### Example 6. Production of the compounds of formulas (VI-XIII).

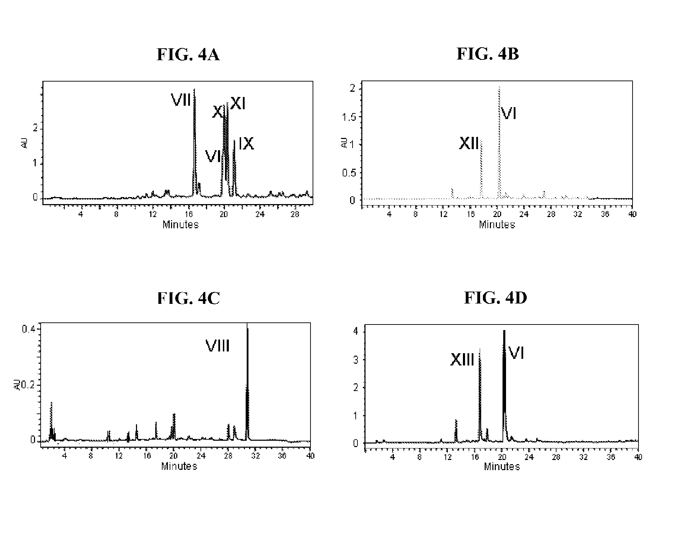

To purify the OVM derivatives, the strains *S*. *albus* (pFL1030), *S. albus* (pFL1031), *S. albus* (pFL1033) and *S. albus* (pFL1146) were cultured in medium R5A using a two-step culture method, as previously described (J. Bacteriol. 1998, 180, 4929-4937). In the production step 8 x 2 litre Erlenmeyer flasks were used, each one containing 400 ml of medium, which were incubated for 5 days. The cultures were centrifuged and filtered, and the supernatant was extracted in the solid phase as described (Chem. Biol. 2002, 9, 519-531). In the case of the *S*. *albus* strain (pFL1031), the supernatant was acidified at pH 4.0 with formic acid before the extraction. The fractions obtained were analysed by HPLC-MS using a chromatographic apparatus coupled to a ZQ4000 mass spectrometer (Waters - Micromass), using acetronitrile and 0.1 % trifluoroacetic acid (TFA) in water as solvents, and a reverse phase column (Symmetry C18, 2.1 x 150 mm, Waters). The samples were eluted with 10% acetonitrile during the first 4 min., followed by a linear gradient 10-88% acetronitrile during 26 min., at a flow of 0.25 ml/min. The detection and spectral characterization of the peaks was performed with a photodiode detector and Empower software (Waters). The MS analyses were performed by electrospray ionization in positive mode with a capillary voltage of 3 kV and cone voltages of 20 and 100 V. Those fractions containing OVM derivatives were vacuum dried. These extracts were dissolved and chromatographed in a µBondapak C18 radial compression column (PrepPak Cartridge, 25 x 100 mm, Waters) or in a preparative column (SunFire Prep C18, 10 x 250 mm, Waters). For each compound, isocratic elutions were optimized with mixtures of acetronitrile or methanol and 0.1 % TFA in water, at 10 ml/min. In all cases, after each purification step, the compounds collected were diluted 4 times with water and were desalinated and concentrated by extraction in solid phase, to be finally freeze-dried. Fig. 4 shows the resulting chromatograms of the four strains used.

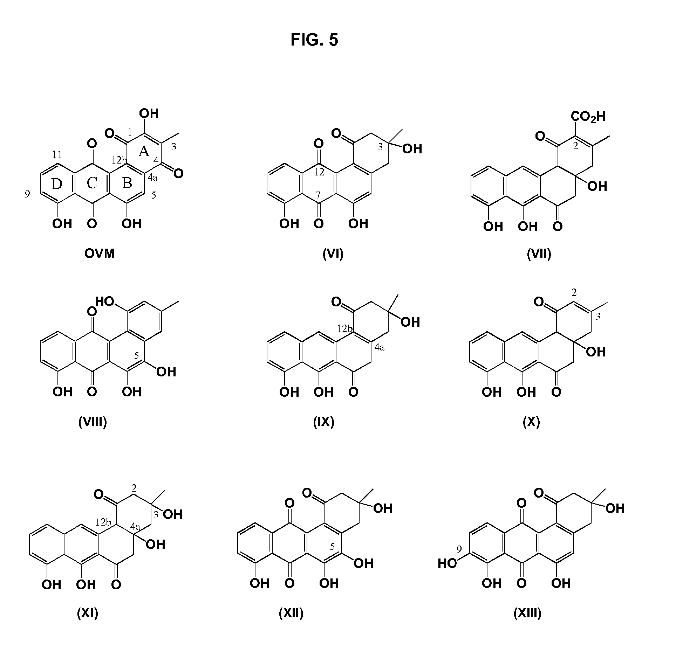

In this way, the following compounds were obtained (Fig. 5). From S. *albus* (pFL1030): 2.0 mg of 5-hydroxy-dehydro-rabelomycin [formula (VIII)]. From *S. albus* (pFL1031): 5.7 mg of prejadomycin-2-carboxylate [formula (VII)], 4.2 mg of rabelomycin [formula (VI)], 17.8 mg of prejadomycin [formula (X)], 21.5 mg of UWM6 [formula (XI)], 3.7 mg of 4a,12b-dehydro-UWM6 [formula (IX)]. From *S*. *albus* (pFL1033): 7.3 mg of 5-hydroxy-rabelomycin [formula (XII)], 37.3 mg of rabelomycin [formula (VI)]. From *S*. *albus* (pFL1146): 13.8 mg of 9-hydroxy-rabelomycin [formula (XIII)], 33.0 mg of rabelomycin [formula (VI)]. As described in Example 7, some of these compounds are already known [formulas (VI, X, XI, XII)], whilst others are produced here for the first time [formulas (VII, VIII, IX, XIII)].

### Example 7. Characterization of formulas (VI), (VII), (VIII), (IX), (X), (XI), XII) and (XIII)

The structures of the compounds of formulas (VI), (VII), (VIII), (IX), (X), (XI), (XII) and (XIII), isolated in the manner described in Example 6, were studied by mass spectrometry (MS) and nuclear magnetic resonance spectrometry (NMR). The ESI-MS spectrum (electrospray ionization-MS) were acquired using a Finnigan LCQ spectrometer. The ionization impact (EI) mass spectrums were measured using a Finnigan PolarisQ spectrometer. The high resolution ionization mass EI spectrums were performed at 25 eV in a JEOL JMS-700T M station (magnetic sector instrument) at a resolution greater than 10000. All NMR data were obtained in *d₆*-DMSO using a Varian Mercury 300 spectrometer, or a Varian Inova 400 MHz spectrometer. All NMR assignments were confirmed by HSQC (heteronuclear single quantum correlation) spectrums and HMBC (heteronuclear multiple bond correlation) spectrums, which permitted the unequivocal assignment of all signals.

The compounds of formulas (VI), (X), (XI) and (XII) were identified as the already known rabelomycin, prejadomycin, UWM6 and 5-hydroxy-rabelomycin, respectively, by the comparison of their NMR data with previously published data *(*Microbiology 2000, 146, 147-154; J. Magn. Reson. 2000, 146, 232-239 ; J. Nat. Prod. 2002, 65, 221-244; WO 02/074800 2002). Rabelomycin [formula (VI)] and the compound UWM6 [formula (XI)] have been previously isolated as products of several biosynthetic routes (J. Antibiot. 1970, 23, 437-441; Microbiology 1996, 142, 123-132; J. Antibiot. 2004, 57, 502-510; ChemBioChem 2005, 6, 1-8; J. Biol. Chem. 2005, 280, 22508-22514*;* Angew. Chem. Int. Ed. 2006, 45, 7842-7846; J. Am. Chem. Soc. 2004, 126, 12262-12263; J. Am. Chem. Soc. 1999, 121, 1786-1794; J. Am. Chem. Soc. 2004, 126, 4496-4497; Antimicrob. Agents Chemother. 2003, 47, 1291-1296). The only existing difference between prejadomycin [formula (X)] and UWM6 [formula (XI)] is the hydration of its 2,3-enoyl bond. The compound UWM6 is unstable and spontaneously becomes rabelomycin [formula (VI)] by dehydration and oxidation (J. Am. Chem. Soc. 1999, 121, 1786-1794).

The compounds produced and described here for the first time are: prejadomycin-2-carboxylate [formula (VII)], 5-hydroxy-dehydro-rabelomycin [formula (VIII)], 4a,12b-dehydro-UWM6 [formula (IX)], and 9-hydroxy-rabelomycin [formula (XIII)]. These compounds have the following physicochemical properties:
**Prejadomycin-2-carboxylate [formula (VII)]:** yellow solid; 1.8 mg/L; Rᵣₑₗ = 18.1 min; maximum in UV (measured with photodiode detector in HPLC): 266 (69%), 280 (100), 407 (29%); (-)-APCI-MS: *m*/*z* 367 ([M-H], 100); (+)-APCI-MS: *m*/z (%) = 369 ([M+H]⁺, 100); HREI-MS *(m*/*z* 368.0834; 368.0896 calculated for C₂₀H₁₆O₇); the data of ¹H and ¹³C NMR are shown in Table 1.
**5-hydroxy-dehydro-rabelomycin [formula (VIII)]:** green solid; 0.6 mg/L; Rᵣₑₗ = 31.1 min; maximum in UV (measured with photodiode detector in HPLC): 224 (44%), 345 (67%), 456 (15 %); (-)-APCI-MS: *m*/*z* (%) = 335 ([M-H], 100); (+)-APCI-MS: *m*/z (%) = 337 ([M+H]⁺, 100); HREI-MS *(m*/z 336.0642; 336.0634 calculated for C₁₉H₁₂O₆); the data of ¹H and ¹³C NMR are shown in Table 2.
**4a,12b-dehydro-UWM6 [formula (IX)]:** yellow solid; 1.2 mg/L; Rᵣₑₗ = 21.2 min; maximum in UV (measured with photodiode detector in HPLC): 241 (45%), 259 (89%), 403 (16%); (-)-APCI-MS: *m*/*z* (%) = 323 ([M-H], 100); (+)-APCI-MS: *m*/*z* (%) = 325 ([M+H]⁺, 100); HREI-MS (*m*/*z* 324.0909; 324.0998 calculated for C₁₉H₁₆O₅); the data of ¹H and ¹³C NMR are shown in Table 3.
**9-hydroxy-rabelomycin [formula (XIII)]:** yellow solid; 4.3 mg/L; Rᵣₑₗ =16.8 min; maximum in UV (measured with photodiode detector in HPLC): 271 (75 %), 286 (100%), 437 (20 %); (-)-APCI-MS: *m*/*z* (%) = 353 ([M-H], 100); (+)-APCI-MS: *m*/*z* (%) = 355 ([M+H]⁺, 100); HREI-MS *(m*/*z* 354.0714; 354.0739 calculated for C₁₉H₁₄O₇); the data of ¹H and ¹³C NMR are shown in Table 4.

In a previous publication, it has been suggested that the compound 4a,12b-dehydro-UWM6 [formula (IX)] could be found in balance with prejadomycin (formula X) through a Michael intramolecular reordering reaction *(*Angew. Chem. Int. Ed. 2006, 45, 7842-7846). It is clear that in said publication the structure of formula IX was only proposed as hypothetical. This application describes for the first time the production and characterization of the compound 4a,12b-dehydro-UWM6 [formula (IX)].

### Example 8. Anti-tumour activity of the compounds of formulas (VI), (VII), (VIII), (IX), (X), (XI), (XII) and (XIII).

The OVM derivatives were tested against a series of cell lines from tumours. Cell growth and viability were quantitatively determined with a colorimetric type test, using the reaction with sulforhodamine B (SRB), according to the technique described by Faircloth et al. (Journal of Tissue and Culture Methods 1988, 11, 201-205). The results are shown in Table 5.

96-well microtiter plates are inoculated with cells (5x10³ cells per well) in aliquots of 195 µl of medium, incubating them for 18 h, in medium without added compound, to allow the cells to adhere to the surface. Next, the compounds to be tested are added, in samples of 5 µl, in a concentration range of 10 to 10⁻⁸ µg/ml, dissolved in DMSO/EtOH (0.2% in PS buffer). After 48 h exposure, the anti-tumour effect is measured using the SRB technique: the cells are fixed by adding 50 µl of cold trichloroacetic acid (50%) (w/v) and it is incubated for 60 min. at 4°C. The plates are washed with deionized water and dried. 100 µl of SRB solution (0.4% w/v in 1% acetic acid) are added to each well, and it is incubated for 10 min. at ambient temperature. The unbound SRB is eliminated, washing with 1% acetic acid. The plates are air dried and the bound colorant is dissolved with Tris buffer. The optical densities are read in an automatic dish spectrophotometer reader at a wavelength of 490 nm.

Table 5 shows the results of the Gl₅₀ (growth inhibition). Most compounds showed a similar anti-tumour activity to that of the OVM. However, the compounds prejadomycin-2-carboxylate [formula (VII)], 4a,12b-dehydro-UWM6 [formula (IX)] and prejadomycin [formula (X)] showed clearly greater activities than that of the OVM, with Gl₅₀ values of submicromolar range. These results suggest that the absence of oxygenation in the C-12 position increases the anti-tumour activity of this class of compounds. Therefore, the elimination of OvmOl oxygenase (responsible for said oxygenation) is useful for the production of more active angucyclines.

**Table 1. NMR data for prejadomycin 2-carboxylate [formula (VII)] in d₆-DMSO (¹H at 400 MHz, ¹³C at 100 MHz).**

| **Position** | δ**_{H} (ppm), multiplicity (*J* in Hz)** | **δ_{c} (ppm)** | **HMBC** |
|---|---|---|---|
| 1 | - | 156.8 | |
| 1-OH | 14.75^{[a]}, 1 H, s (br) | - | |
| 2 | - | 131.3 | |
| 2-COOH | 9.85 ^{[a]}, 1 H, s (br) | 175.2 | |
| 3 | - | 164.4 | |
| 4 | 2.62, 2.59, 2H, dd, 14.8 | 40.6 | 2,12b |
| 4a | - | 71.6 | |
| 4-OH | 3.96^{[a]}, 1 H, s | | |
| 5 | 2.94, 2.35, 2H, dd, 17.4 | 43.8 | 4a, 6a, 12b |
| 6 | - | 202.7 | |
| 6a | - | 111.1 | |
| 7 | - | 166.9 | |
| 7a | - | 118.2 | |
| 7-OH | 15.54 ^{[a]}, 1 H, s (br) | - | |
| 8 | - | 157.2 | |
| 8-OH | 9.42^{[a]}, 1 H, s | - | |
| 9 | 6.88, 1 H, d, 8.2 | 108.9 | 7a, 811 |
| 10 | 7.56, 1 H, t, 8.2 | 132.7 | 8, 9, 11a |
| 11 | 7.25, 1 H, d, 8.2 | 112.6 | 7a, 9, 10 |
| 11a | - | 138.7 | |
| 12 | 6.85, 1 H, s | 119.5 | 6a, 11a |
| 12a | - | 135.25 | |
| 12b | - | 101.2 | |
| 13-CH₃ | 1.96, 3H, s | 21.6 | 2,3 |

| | | | |
|---|---|---|---|
| ^{[a]} Interchangeable with D₂O. | | | |

**Table 2. NMR spectrums for 5-hydroxy-dehydro-rabelomycin [formula (VIII)] in d₆-DMSO (¹H at 400 MHz, ¹³C at 100 MHz).**

| **Position** | δ**_{H} (ppm), multiplicity (*J* in Hz)** | **δ_{c} (ppm)** |
|---|---|---|
| 1 | - | 158.5 |
| 2 | 6.82, d, 1.9, 1 H | 117.5 |
| 3 | - | 139.3 |
| 4 | 7.54, 1 H, d, 1.9 | 113.1 |
| 4a | - | 129.9 |
| 5 | - | 151.2 |
| 6 | - | 154.9 |
| 6-OH | 11.60, brs, 1 H | - |
| 6a | - | 110.5 |
| 7 | - | 192.9 |
| 7a | - | 115.2 |
| 8 | - | 160.5 |
| 8-OH | 12.56, brs, 1 H | - |
| 9 | 7.30, d, 7.5, 1 H | 122.7 |
| 10 | 7.78, t, 7.5, 1 H | 137.7 |
| 11 | 7.69, d, 7.5, 1 H | 119.5 |
| 11a | - | 136.5 |
| 12 | - | 187.6 |
| 12a | - | 137.3 |
| 12b | - | 118.0 |
| 3-CH₃ | 2.39, s, 3H | 21.2 |

**Table 3. NMR spectrums for 4a,12b-dehydro-UWM6 [formula (IX)] in d₆-DMSO (¹H at 400 MHz, ¹³C at 100 MHz).**

| **Position** | δ**_{H} (ppm), multiplicity (*J* in Hz)** | δ**_{c} (ppm)** | **HMBC** |
|---|---|---|---|
| 1 | - | 207.8 | |
| 1-OH | - | - | |
| 2 | 2.42, 1.88, 2H, d, 13.8 | 52.9 | 4a, 12b |
| 2-COOH | - | - | |
| 3 | - | 75.8 | |
| 4 | 2.66, 1.98, 2H, dd, 14.7 | 42.3 | 3, 13 |
| 4a | - | 142.8 | |
| 4-OH | - | - | |
| 5 | 3.08, 2.83, 2H, dd, 16.7 | 51.3 | 6a, 12b |
| 6 | - | 202.5 | |
| 6a | - | 111.9 | |
| 7 | - | 165.9 | |
| 7a | - | 116.6 | |
| 7-OH | 15.62, 1 H, s (br) | - | |
| 8 | - | 157.3 | |
| 8-OH | 9.88, 1 H, s | - | |
| 9 | 6.87, 1 H, d, 8.9 | 109.0 | 7a, 11 |
| 10 | 7.52, 1 H, t, 8.9 | 133.8 | 8, 11a |
| 11 | 7.25, 1 H, d, 8.9 | 113.4 | 7a, 9, 12 |
| 11a | - | 139.9 | |
| 12 | 6.76, 1 H, s | 118.2 | 6a, 7a |
| 12a | - | 135.2 | |
| 12b | - | 137.6 | |
| 13-CH₃ | 1.11, 3H, s | 29.8 | 2,3,4 |

**Table 4. NMR spectrums for 9-hydroxy-rabelomycin [formula (XIII)] in d₆-DMSO (¹H at 400 MHz, ¹³C at 100 MHz).**

| **Position** | δ**_{H} (ppm), multiplicity** (***J* in Hz)** | δ**_{c} (ppm)** |
|---|---|---|
| 1 | - | 196.0 |
| 2 | 2.94, d, 13.1, 1 H; | 53.3 |
| | 2.67, d, 13.1, 1 H | |
| 3 | - | 71.1 |
| 4 | 3.11, d, 15.3, 1 H; | 43.6 |
| | 2.98, d, 15.3, 1 H | |
| 4a | - | 150.0 |
| 5 | 7.07, s, 1H | 121.2 |
| 6 | - | 162.2 |
| 6-OH | 11.99, brs, 1 H | - |
| 6a | - | 116.8 |
| 7 | - | 191.8 |
| 7a | - | 115.7 |
| 8 | - | 151.9 |
| 8-OH | 11.95, brs, 1 H | |
| 9 | - | 152.21 |
| 10 | 7.20, d, 8.0, 1 H | 120.7 |
| 11 | 7.40, d, 8.0, 1 H | 120.0 |
| 11a | - | 125.5 |
| 12 | - | 181.6 |
| 12a | - | 137.9 |
| 12b | - | 128.9 |
| 3-CH₃ | 1.28, s, 3H | 29.6 |

**Table 5 Test of the anti-tumour activity of OVM derivatives against tumour cell lines. Also includes the data obtained with OVM, as reference. The numerical values make reference to the Gl₅₀ (µM), or concentration at which the compound tested inhibits 50% of the cell growth in comparison with untreated cells. formula VI: rabelomycin; formula VII: prejadomycin 2-carboxylate; formula VIII: 5-hydroxy-dehydro-rabelomycin; formula IX: 4a,12b-dehydro-UWM6; formula X: prejadomycin; formula XI: UWM6; formula XII: 5-hydroxy-rabelomycin; formula XIII: 9-hydroxy-rabelomycin.**

| Cell line (cancer type) | compounds | | | | | | | | |
|---|---|---|---|---|---|---|---|---|---|
| | OVM | VI | VII | VIII | IX | X | XI | XII | XIII |
| MDA-MB-231 (breast) | 5.6 | 6.80 | 0.38 | 2.56 | 0.65 | 0.24 | 24.8 | 13.3 | 7.06 |
| A549 (lung) | 8.7 | 5.32 | 0.60 | 6.24 | 0.77 | 0.29 | >100 | 7.62 | 3.10 |
| HT29 (colon) | 6.9 | 10.9 | 1.17 | 8.03 | 1.85 | 1.29 | >100 | 12.4 | 14.1 |

## Claims

1. A compound with any of formulas (I), (II), (III), (IV) or (V) 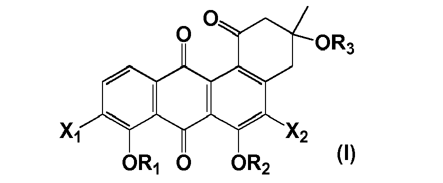 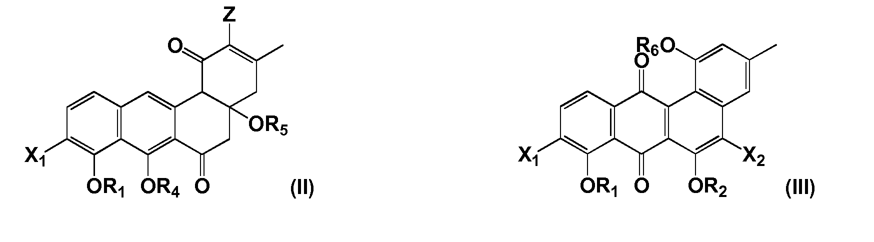 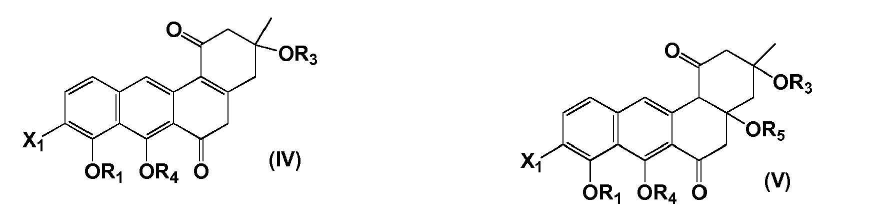 where
R₁, R₂, R₃, R₄, R₅ and R₆ are, each one and independently, hydrogen or a protector group, which may consist of an alkyl group, a cycloalkyl group, a heterocyclic cycloalkyl group, a hydroxyalkyl group, a halogenated alkyl group, an alkoxyalkyl group, an alkenyl group, an alkynyl group, an aryl group, a heterocyclic aryl group, an alkylaryl group, an ester group, a carbonate group, a carboxylic acid group, an aldehyde group, a ketone group, a urethane group, a silyl group, a sulfoxide group or a combination thereof,
X₁ and X₂ are, each one and independently hydrogen, a hydroxyl group or an -OR₁ group, where R₁ is a protector group according to the previous definition,
Z is hydrogen or a group chosen from the following groups: a carboxylic acid group, an ester group, a carbonate group, a carbamate group, a urethane group, a hydroxyl group, an alkyl group, a cycloalkyl group, a heterocyclic cycloalkyl group, a hydroxyalkyl group, a halogenated alkyl group, an alkoxyalkyl group, an alkenyl group, an alkynyl group, an aryl group, a heterocyclic aryl group, an alkylaryl group, an aldehyde group or a ketone group.

2. The compound of claim 1, with formula (VII):

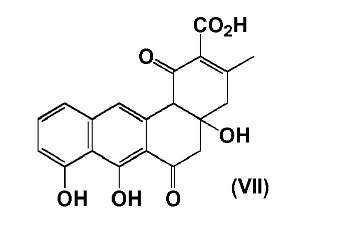

3. The compound of claim 1, with formula (VIII):

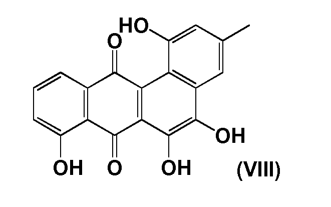

4. The compound of claim 1, with formula (IX):

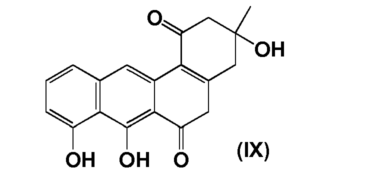

5. The compound of claim 1, with formula (XIII):

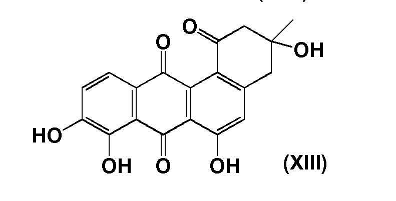

6. Bacterial strains derived from *Streptomyces albus,* **characterized in that** each one of said strains has an additional nucleic acid which encodes active enzymes for the biosynthesis of oviedomycin derivatives, in accordance with any of formulas (I), (II), (III), (IV) or (V), these enzymes not being present in *Streptomyces albus.*

7. A bacterial strain according to claim 6, **characterized in that** said strain is *Streptomyces albus* (pFL1030).

8. A bacterial strain of claim 6, **characterized in that** said nucleic acid is the plasmid pFL1030, which encodes active enzymes for the biosynthesis of the compound of formula (VIII) and of its biosynthetic intermediaries.

9. A bacterial strain according to claim 6, **characterized in that** said strain is *Streptomyces albus* (pFL1031).

10. A bacterial strain of claim 6, **characterized in that** said nucleic acid is the plasmid pFL1031, which encodes active enzymes for the biosynthesis of the compounds of formula (VI), (VII), (IX) and (X) and of their biosynthetic intermediaries.

11. A bacterial strain according to claim 6, **characterized in that** said strain is *Streptomyces albus* (pFL1146).

12. A bacterial strain of claim 6, **characterized in that** said nucleic acid is the plasmid pFL1146, which encodes active enzymes for the biosynthesis of the compounds of formula (VI) and (XIII) and of their biosynthetic intermediaries.

13. A method to obtain the bacterial strains of claim 6, which comprises the introduction of a nucleic acid in *Streptomyces albus* or in a strain derived from *Streptomyces albus.*

14. The method of claim 13, which comprises the introduction of plasmid pFL1030 in *Streptomyces albus.*

15. The method of claim 13, which comprises the introduction of plasmid pFL1031 in *Streptomyces albus.*

16. The method of claim 13, which comprises the introduction of plasmid pFL1046 in *Streptomyces albus.*

17. A method for producing oviedomycin derivatives of any of formulas (I), (II), (III), (IV) or (V), which comprises:
a) incubating a bacterial strain of any of claims 6 to 12 to produce a composition including an oviedomycin derivative in accordance with any of formulas (I), (II), (III), (IV) or (V); and
b) isolating an oviedomycin derivative from the composition produced in step (a).

18. A method according to claim 17, **characterized in that** the bacterial strain is *Streptomyces albus* (pFL1030).

19. A method according to claim 17, **characterized in that** the bacterial strain is *Streptomyces albus* (pFL1031).

20. A method according to claim 17, **characterized in that** the bacterial strain is *Streptomyces albus* (pFL1046).

21. A method according to claim 17, **characterized in that** the oviedomycin derivative is rabelomycin [formula (VI)].

22. A method according to claim 17, **characterized in that** the oviedomycin derivative is prejadomycin 2-carboxylate [formula (VII)].

23. A method according to claim 17, **characterized in that** the oviedomycin derivative is 5-hydroxy-dehydro-rabelomycin [formula (VIII)].

24. A method according to claim 17, **characterized in that** the oviedomycin derivative is 4a,12b-dehydro-UWM6 [formula (IX)].

25. A method according to claim 17, **characterized in that** the oviedomycin derivative is prejadomycin [formula (X)].

26. A method according to claim 17, **characterized in that** the oviedomycin derivative is UWM6 [formula (XI)].

27. A method according to claim 17, **characterized in that** the oviedomycin derivative is 9-hydroxy-rabelomycin [formula (XIII)].

28. An oviedomycin derivative compound defined by any of formulas (I), (II), (III), (IV) or (V) that can be obtained according to the method of claim 17.

29. An oviedomycin derivative compound defined by any of formulas (I), (II), (III), (IV) or (V) and **characterized in that** it is produced by a bacterial strain of any of claims 6 to 12.

30. An oviedomycin derivative compound in accordance with any of formulas (I), (II), (III), (IV) or (V); **characterized in that** it is produced by the *Streptomyces albus* strain (pFL1030) of claim 7.

31. An oviedomycin derivative compound in accordance with any of formulas (I), (II), (III), (IV) or (V); **characterized in that** it is produced by the *Streptomyces albus* strain (pFL1031) of claim 9.

32. An oviedomycin derivative compound in accordance with any of formulas (I), (II), (III), (IV) or (V); **characterized in that** it is produced by the *Streptomyces albus* strain (pFL1146) of claim 11.

33. A pharmaceutical preparation comprising, among other components, a therapeutically effective quantity of a compound of claim 1, or a pharmaceutically acceptable salt, solvate or prodrug thereof, together with one or more excipients and diluents.

34. A pharmaceutical preparation comprising, among other components, a therapeutically effective quantity of a compound of claim 29, or a pharmaceutically acceptable salt, solvate or prodrug thereof, together with one or more excipients and diluents.

35. Use of a compound of any of formulas (I), (II), (III), (IV) or (V) according to claim 1, or a pharmaceutically acceptable salt, solvate or prodrug thereof in the manufacturing of a drug to treat cancer in a subject diagnosed with said disease.

36. Use according to claim 35, where the subject is a human being.

37. Use of the pharmaceutical preparation of claim 33, to prepare a drug to treat cancer in a subject diagnosed with said disease.

38. Use according to claim 37, where the subject is a human being.

39. Use of the pharmaceutical preparation of claim 34, to prepare a drug to treat cancer in a subject diagnosed with said disease.

40. Use according to claim 39, where the subject is a human being.
